# EUROPEAN PATENT APPLICATION

(11) **EP 1 473 043 A1**
(43) Date of publication of application: **03.11.2004**
(21) Application number: 03009587.1
(22) Date of filing: 29.04.2003
(51) Int. Cl.: A61K 45/06, A61K 31/496, A61K 31/56, A61P 35/00

(54) **Pharmaceutical combination for the treatment of diseases involving cell proliferation, migration or apotosis of myeloma cells, or angiogenesis**

(71) Applicant: Boehringer Ingelheim Pharma GmbH & Co.KG, 55218 Ingelheim am Rhein (DE)
(72) Inventor: Stefanic, Martin, Dr., 88447 Warthausen (DE); Munzert, Gerd, Dr., 89075 Ulm (DE); Hilberg, Frank, Dr., A-1050 Wien (AT)

(57) **Abstract**

The present invention relates to a pharmaceutical combination for the treatment of diseases which involves cell proliferation, migration or apoptosis of myeloma cells, or angiogenesis. The combination comprises the co-administration of a protein tyrosine kinase receptor antagonist and of a steroid.

## Description

### Field of the Invention

This invention relates to: A method for the treatment of diseases involving cell proliferation, migration or apoptosis of myeloma cells, or angiogenesis, which method comprises co-administration of effective amounts of a protein tyrosin kinase receptor antagonist and a steroid, to a person in need of such treatment; Suitable pharmaceutical compositions comprising a specific protein tyrosin kinase receptor antagonist and a steroid, as a combined preparation for simultaneous, separate or sequential use in the treatment of diseases involving cell proliferation, migration or apoptosis of myeloma cells, or angiogenesis, and especially for inhibiting tumour growth, survival and metastasis; The combined use of a protein tyrosin kinase receptor antagonist and a steroid, for the manufacture of a pharmaceutical combination preparation for the treatment of said diseases.

### Background of the Invention

In the last decade, the biological activity of several types and sub-types of the protein tyrosin kinase receptor family have been characterised such as, for example, the epidermal growth factor receptor EGFR and its subtypes ErbB-2 and ErbB-4 (Brignola et al., Journal of Biological Chemistry, Vol. 277, No.2, pp. 1576-1585, 2002) or the vascular endothelial growth factor receptors VEGFR 1-3 together with its ligand VEGF and its four sub-types known to date (Jung et al., European Journal of Cancer, Vol. 38, pp. 1133-1140, 2002). Similar studies reported on previous reports showing that the overexpression of some of these receptors is implicated in multiple forms of cancer. For example, studies have provided evidence that the epidermal growth factor EGF acts as a growth factor in tumours, and that the vascular endothelial growth factor VEGF is one of the most common mediators of tumor angiogenesis, which is essential for the growth and metastasis of solid tumours. Inhibitors of the receptors have thus been and are still evaluated for cancer therapy (see for example the article of Cerrington et al. In Advances in Cancer Research, Academic Press 2000, pp. 1-38).

For cancer therapy, recent studies have suggested to combine several receptor antagonists together, or in further combination with a chemotherapeutic agent or radiation. For example, WO 02/070008 suggests the combination of a VEGFR and an EGFR receptor antagonist, optionally together with radiation or a chemotherapeutic agent, for the inhibition of tumour growth.

A large number of protein tyrosine kinase receptor antagonists are currently in clinical development for the treatment of cancer (see for example the Expert Opinion Review of Laid & Cherrington in Expert Opin. Invest. Drugs, Vol. 12, No. 1, pp. 51-64, 2003). However, proof of efficacy for these substances, used alone or with other cancer therapies, in the treatment of oncological diseases, has so far not been achieved, either because of a lack of additional benefit over the standard therapy or because of the discovery of unacceptable side-effects.

For example, it has been recently published that an angiogenesis inhibitor which has already been clinically tested, also in conjunction with chemotherapy, namely the inhibitor with code name SU5416, developed by Pharmacia for the treatment of cancer, was associated with disturbing side effect, namely thromboembolic events (Ken Garber and Ann Arbor, Nature Biotechnology, Vol. 20, pp. 1067-1068, Nov. 2002).

In other respects, it is already known to use steroids in the treatment of some cancers. The reasons why steroids are used in the treatment of some cancers and the effects obtained with steroids in the treatment of cancer depends on the type of cancer to be treated.

In the treatment of solid tumors, steroids are in first line used to control the symptoms. In the case of brain metastasis, they belong to the standard therapy for reducing oedema. They are also used to control the inflammation which surrounds the tumor lesions.

In the treatment of haematologic malignant neoplasias of lymphatic cell lines (ALL, non-Hodgkin lymphoma, myeloma), due to their cytolytic effect, steroids are used as a real anti-tumor therapy, alone or in combination with classical chemotherapeutic agents.

The naturally occuring steroid tetrahydrocortisol, the synthetic cyclodextrin derivative β-cyclodextrine tetradecasulfate and the tetracycline derivative minocycline, due to their antiangiogenic activity, have been suggested for a combination treatment with cytotoxic standard anticancer therapies, such as platinum, melphalan, cyclophosphamide, adriamycin, bleomycin or radiation based therapies (Teicher et al., Cancer research, Vol. 52, pp. 6702-6704, 1992).

The steroid dexamethasone has also been tested as primary treatment of multiple myeloma (Dimopoulos et al., Blood, Vol. 80(4), pp. 887-890, 1992).

Evaluation studies of combination therapies using dexamethasone and thalidomide, a substance known for its activity as TNF-α synthesis inhibitor and cytokine antagonist, have been disclosed recently. These studies focussed on previously untreated multiple myeloma (Weber et al., Journal of Clinical Oncology, Vol. 21, No. 1, pp. 16-19, 2003), newly diagnosed myeloma (Rajkumar et al., Journal of Clinical Oncology, Vol. 20, No. 21, pp. 4319-4323, 2002) and multiple myeloma after intensive chemotherapy (Ann. Oncol., Vol. 13, No. 7, pp. 1116-1119, 2002).

There is however still a need for new and efficient combination therapies for the treatment of diseases in which cell proliferation, migration or apoptosis of myeloma cells, or angiogenesis are involved.

These diseases may as well be of oncological nature, which includes all types of malignant neoplasias or cancers, or of non-oncological nature, such as diabetic retinopathy, rheumatoid arthritis or psoriasis.

### Summary of the Invention

It has now been found that co-administration of an effective amount of a protein tyrosine kinase receptor antagonist, or of a polymorph, metabolite or pharmaceutically acceptable salt thereof, with an effective amount of a steroid, provides unexpected advantages in the treatment of diseases in which cell proliferation, migration or apoptosis of myeloma cells, or angiogenesis are involved, to a person in need of such treatment, with high efficacy, in comparison to administration of any of these substances alone.

Further it has been found that the diseases which can be treated by the combination in accordance with the present invention are all kind of diseases in which cell proliferation, migration or apoptosis of myeloma cells, or angiogenesis are involved, which can be of oncological nature such as all types of malignant neoplasias or cancers, or of non-oncological nature, such as diabetic retinopathy, rheumatoid arthritis, or psoriasis.

Further it has been found that the combination treatment in accordance with the present invention is especially efficient for inhibiting tumour growth, survival and metastasis.

Further it has been found that the combination treatment in accordance with the present invention is especially efficient with selected active substances, selected dosages and selected dosage forms.

Thus, the present invention provides a method for the treatment of diseases involving cell proliferation, migration or apoptosis of myeloma cells, or angiogenesis, which method comprises co-administration of effective amounts of a protein tyrosine kinase receptor antagonist, or of a polymorph, metabolite or pharmaceutically acceptable salt thereof, and of a steroid, to a person in need of such treatment.

The protein tyrosine kinase receptor antagonist used in the method in accordance with the present invention is preferably an antagonist of at least one receptor selected from VEGFR 1 to 3, PDGFR α and β, FGFR1, 2 and 3, EGFR, HER2, IGF1R, HGFR, c-Kit.

The protein tyrosine kinase receptor antagonist may further be an antagonist of at least one complex of a cyclin dependent kinase with its specific cyclin or with a viral cyclin, an antagonist of a src-tyrosine kinase family member, or an inhibitor of the paracrine IL-6 secretion.

In one preferred embodiment, the protein tyrosine kinase receptor antagonist is 3-Z-[1-(4-(N-((4-methyl-piperazin-1-yl)-methylcarbonyl)-N-methyl-amino)-anilino)-1-phenylmethylene]-6-methoxycarbonyl-2-indolinone , or a polymorph, metabolite or pharmaceutically acceptable salt thereof.

In another preferred embodiment, the protein tyrosine kinase receptor antagonist is the monoethanesulfonate salt of 3-Z-[1-(4-(N-((4-methyl-piperazin-1-yl)-methylcarbonyl)-N-methyl-amino)-anilino)-1-phenyl-methylene]-6-methoxycarbonyl-2-indolinone.

The steroid used in the method in accordance with the present invention is preferably selected from prednisone, prednisolone, methylprednisolone, dexamethasone, budenoside, fluocortolone and triamcinolone.

In one preferred embodiment, the steroid is dexamethasone.

In one embodiment, the disease treated in the method in accordance with the present invention is preferably an oncological disease. In a preferred embodiment, the disease is multiple myeloma.

In another embodiment, the disease treated in the method in accordance with the present invention is preferably a non-oncological disease selected from diabetic retinopathy, rheumatoid arthritis or psoriasis.

Thus, the beneficial efficacy of the methods in accordance with the invention are mainly based on the additive and synergistic effects of the combined treatment.

The unexpected advantages mentioned above may be due to a more efficient apoptosis induction by the steroid, once the constitutively active survival signal of the protein tyrosin kinase receptor, mediated by the tumour, is inhibited by the protein tyrosine kinase receptor antagonist.

In the case of the use of an antagonist of protein tyrosine kinase receptors or an inhibitor of other mediators involved in angiogenesis, such as for example vascular endothelial growth factors (VEGF), vascular permeability factors, the basic fibroblast growth factor (bFGF), interleukin-6 (IL-6) or interleukin-8 (IL-8), the epidermal growth factor (EGF) or the platelet-derived growth factor (PDGF), one of the advantages of the method and composition in accordance with the present invention lies in a targeting of the treatment to tumor-associated vasculature rather than, or together with, the tumour itself, in order to cut the energy supply of cancerous cells.

A further advantage is that an induction or reinstatement of steroid sensitivity is expected in patients treated with the combination of a steroid and a VEGFR antagonist, especially in the case of patients suffering from refractory multiple myeloma.

According to the present invention, a synergistic combined preparation is meant to comprise an amount of the protein tyrosine kinase antagonist, or of a polymorph, metabolite or pharmaceutically acceptable salt of this active compound, and an amount of the steroid, wherein the amount of the individual agent alone is insufficient to achieve the therapeutic effect achieved by the administration of the combination of said agents, and wherein the combined effects of the amounts of the therapeutic agents is greater than the sum of the therapeutic effects achievable with the amounts of the individual therapeutic agents.

Viewed from a different aspect, the present invention also relates to a pharmaceutical combination for the treatment of diseases in which cell proliferation, migration or apoptosis of myeloma cells, or angiogenesis are involved, comprising a selected specific protein tyrosine kinase receptor antagonist and a steroid, as a combined preparation for simultaneous, separate or sequential use in treatment of said diseases, optionally together with one or more pharmaceutically acceptable diluents and/or carriers.

Viewed from a different aspect, the present invention also relates to a pharmaceutical combination preparation kit for the treatment of diseases involving cell proliferation, migration or apoptosis of myeloma cells, or angiogenesis, comprising a therapeutically effective amount of a protein tyrosine kinase receptor antagonist, or of a polymorph, metabolite or pharmaceutically acceptable salt thereof, and a therapeutically effective amount of a steroid, characterised in that the protein tyrosine kinase receptor antagonist is comprised within a first compartment and the steroid is comprised within a second compartment, such that the administration to a patient in need thereof can be simultaneous, separate or sequential.

In one embodiment in accordance with the present invention, in each compartment of the pharmaceutical combination preparation kit, each active substance is formulated for an oral administration.

Viewed from a further aspect, the present invention thus also provides the use of a protein tyrosine kinase receptor antagonist in combination with a steroid, for the manufacture of a pharmaceutical combination preparation for the treatment of the diseases or indications mentioned hereinbefore.

### Detailed Description of the Invention

As already mentioned hereinbefore, the diseases which can be treated by the combination in accordance with the present invention are all kind of diseases in which cell proliferation, migration or apoptosis of myeloma cells, or angiogenesis are involved, which can be of oncological nature such as all types of malignant neoplasias or cancers, or of non-oncological nature, such as diabetic retinopathy, rheumatoid arthritis, or psoriasis. Among cancers, selected specific target indications are solid tumours, such as urogenital cancers (such as prostate cancer, renal cell cancers, bladder cancers), gynecological cancers (such as ovarian cancers, cervical cancers, endometrial cancers), lung cancer, gastrointestinal cancers (such as colorectal cancers, pancreatic cancer, gastric cancer, oesophageal cancers, hepatocellular cancers, cholangiocellular cancers), head and neck cancer, malignant mesothelioma, breast cancer, malignant melanoma or bone and soft tissue sarcomas, and haematologic neoplasias, such as multiple myeloma, acute myelogenous leukemia, chronic myelogenous leukemia, myelodysplastic syndrome and acute lymphoblastic leukemia.

The combination treatment in accordance with the present invention is especially efficient for inhibiting tumour growth, survival and metastasis.

As already mentioned hereinbefore, the protein tyrosine kinase receptor antagonists that can be used in the context of the present invention include all substances that inhibit the stimulation or activation of a protein tyrosine kinase receptor by a protein tyrosine kinase receptor ligand. In the case of a protein tyrosine kinase receptor belonging to the family of the growth factor receptors, such inhibition of stimulation or activation inhibits the growth of cells that express the receptor.

Some examples of growth factor receptors involved in tumorigenesis are the receptors for epidermal growth factor (EGFR), vascular endothelial growth factors (VEGFRs), platelet-derived growth factor (PDGFR), insulin-like growth factor (IGFR), nerve growth factor (NGFR), and fibroblast growth factor (FGFR).

By inhibition of stimulation or activation of protein tyrosine kinase receptor is meant any decrease in the activation of the receptor, which need not completely prevent or stop activation of the receptor.

Moreover, inhibition of the receptor stimulation or activation, as defined by the present invention, means inhibition resulting from interaction of the antagonist and the receptor or its ligand. By interaction is meant sufficient physical or chemical interaction between the antagonist and the receptor, such that protein tyrosin kinase activity is inhibited. One of skill in the art would appreciate that examples of such chemical interactions, which include association or bonding, are known in the art and include covalent bonding, ionic bonding, hydrogen bonding, etc., between the antagonist and the receptor or its ligand.

Increased protein tyrosine kinase receptor stimulation or activation can result from higher levels of ligand, receptor gene amplification, increased transcription of the receptor or mutations that cause unregulated receptor signaling. Amplification of the gene encoding the receptor results in an increased number of ligands binding to the receptor, which can further stimulate cell proliferation. The protein tyrosine kinase receptor may also be overexpressed in the absence of gene amplification, presumably through mutations that increase its transcription, mRNA translation, or stability of the protein. Protein tyrosine kinase receptor mutants of the EGFR type have already been identified in gliomas, non-small cell lung carcinomas, ovarian carcinomas and prostate carcinomas, that have a constitutively active protein tyrosin kinase, suggesting a role for high-level EGFR activity rather than EGFR overexpression in these cancers (see for example Pedersen et al., Ann. Oncol., Vol. 12(6), pp. 745-60, 2001).

In one embodiment in accordance with the present invention, the protein tyrosine kinase receptor antagonist inhibits the binding of the protein tyrosine kinase receptor to its ligand.

Binding of a ligand to an external, extracellular domain of the receptor stimulates receptor dimerization, autophosphorylation of the receptor, activation of the receptor's internal, cytoplasmic protein tyrosin kinase domain, and initiation of multiple signal transduction pathways involved in regulation of DNA synthesis, cell division, vasculogenesis or angiogenesis. The inhibition produced by the presence of the antagonist will consequently reduce this stimulation.

In another embodiment in accordance with the present invention, the protein tyrosine kinase receptor antagonist binds directly to the receptor. The antagonist can bind externally to the extracellular portion of the receptor, which may or may not inhibit binding of the ligand, or internally to the protein tyrosine kinase domain. Examples of such antagonists include, without limitation, biological molecules, such as antibodies (and functional equivalents thereof) specific for the receptor, and synthetic kinase inhibitors that act directly on the cytoplasmic domain of the receptor, such as the so-called "small molecule tyrosine kinase inhibitors". A non-exhaustive list of small molecule tyrosine kinase inhibitors can be found in the review article of Laid & Cherrington, Expert Opinion Invest. Drugs, Vol. 12, No. 1, 2003, the content of which is incorporated herein by reference.

Additional protein tyrosine kinase receptor antagonists can easily be determined using well-known methods. The receptor antagonists to be used in the present invention inhibit the protein tyrosin kinase activity of the receptor, which generally involves phosphorylation events. Accordingly, phosphorylation assays may for example be useful in determining antagonists useful in the context of the present invention. In addition, methods specific for detection of the receptor expression can be utilized. These include immunohistochemistry for detection of protein expression, fluorescence in situ hybridization for detection of gene amplification, competitive radioligand binding assays, solid matrix blotting techniques, such as Northern and Southern blots, reverse transcriptase polymerase chain reaction and ELISA.

In one embodiment in accordance with the present invention, the protein tyrosine kinase receptor antagonist is preferably an antagonist of at least one receptor selected from VEGFR 1 to 3, PDGFR α and β, FGFR1,2 and 3, EGFR, HER2, IGF1R, HGFR. The protein tyrosine kinase receptor antagonist may further be an antagonist of at least one complex of a cyclin dependent kinase with its specific cyclin or with a viral cyclin, an antagonist of one of the src-tyrosine kinase family members, or an inhibitor of the paracrine IL-6 secretion.

In a further embodiment in accordance with the present invention, the combination of the active substances is intended for the treatment of oncological diseases involving angiogenesis.

Tumor angiogenesis plays an important role in the progression of human malignancies. Inhibition of this process is thought to be an excellent point of therapeutic intervention in the treatment of cancer. Signal transduction through the vascular endothelial growth factor receptor 2 (VEGFR-2) has been shown to play a pivotal role in the proliferation, survival and migration of endothelial cells in tumor angiogenesis.

In this matter, a potent and orally available low molecular weight inhibitor of VEGFR-2 has been developed as a new cancer therapeutic agent.

This VEGFR receptor antagonist is 3-Z-[1-(4-(N-((4-methyl-piperazin-1-yl)-methylcarbonyl)-N-methyl-amino)-anilino)-1-phenyl-methylene]-6-methoxycarbonyl-2-indolinone, and most preferably the monoethanesulfonate salt of this compound.

3-Z-[1-(4-(N-((4-methyl-piperazin-1-yl)-methylcarbonyl)-N-methyl-amino)-anilino)-1-phenyl-methylene]-6-methoxycarbonyl-2-indolinone is disclosed, for example, in WO 01/27081, as examplified compound number 473. The monoethanesulfonate salt of 3-Z-[1-(4-(N-((4-methyl-piperazin-1-yl)-methylcarbonyl)-N-methyl-amino)-anilino)-1-phenyl-methylene]-6-methoxycarbonyl-2-indolinone is disclosed, for example, in unpublished German patent application DE 102 33 500.1.

In accordance with what is disclosed in DE 102 33 500.1, the monoethanesulfonate salt of 3-Z-[1-(4-(N-((4-methyl-piperazin-1-yl)-methylcarbonyl)-N-methyl-amino)-anilino)-1-phenyl-methylene]-6-methoxycarbonyl-2-indolinone has the following chemical structure:

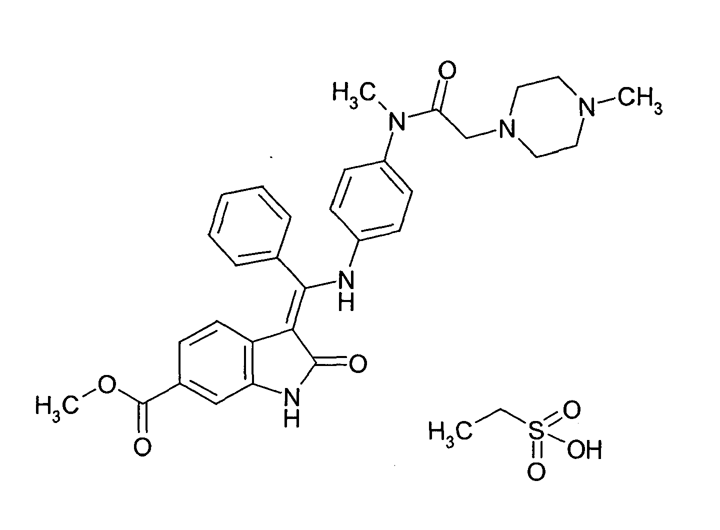

This compound may be selectively obtained by a suitable choice of manufacturing conditions, preferably in its crystalline hemihydrate form.

The compound is characterised by a melting point of T = 305 ± 5°C (determined by DSC = Differential Scanning Calorimetry, using a Mettler-Toledo DSC82 apparatus; heating rate: 10 K/min).

For the manufacture of the monoethanesulfonate salt of 3-Z-[1-(4-(N-((4-methyl-piperazin-1-yl)-methylcarbonyl)-N-methyl-amino)-anilino)-1-phenyl-methylene]-6-methoxycarbonyl-2-indolinone, a procedure in accordance with the following may be used.

The starting material used to prepare the monoethanesulfonate salt of 3-Z-[1-(4-(N-((4-methyl-piperazin-1-yl)-methylcarbonyl)-N-methyl-amino)-anilino)-1-phenyl-methylene]-6-methoxycarbonyl-2-indolinone may be the free base 3-Z-[1-(4-(N-((4-methyl-piperazin-1-yl)-methylcarbonyl)-N-methyl-amino)-anilino)-1-phenyl-methylene]-6-methoxycarbonyl-2-indolinone, which may be obtained in accordance with a method known from the prior art and described, for example, in WO 01/27081.

Thus, in a first step and in accordance with what is described in WO 01/27081, 3-Z-[1-(4-(N-((4-methyl-piperazin-1-yl)-methylcarbonyl)-N-methyl-amino)-anilino)-1-phenyl-methylene]-6-methoxycarbonyl-2-indolinone is prepared as follows.
10.5 g (30.0 mmol) 1-acetyl-3-(1-ethoxy-1-phenylmethylene)-6-methoxycarbonyl-2-indolinone (prepared as described in WO 01/27081) and 8.60 g (33.0 mmol) N-[(4-methyl-piperazin-1-yl)-methylcarbonyl]-N-methyl-p-phenylendiamine (prepared as described in WO 01/27081) are dissolved in 80 ml dimethylformamide and mixed for 1 hour at 80°C. After cooling, 6.50 ml piperidine is added and the whole is further mixed for 2 hours at room temperature. Water is added, the liquid over the resulting precipitate is sucked up, and the precipitate is washed again with a low quantity of water. The residue is suspended in 200 ml methanol, the liquid is sucked up, and the remaining residue washed with cold water and diethylether. The resulting product is vacuum dried at 110 °C.

| | |
|---|---|
| Recovered product: | 12.4 g (77% of theoretical value) |
| IR-spectroscopy: | 1610, 1655, 1711 cm⁻¹ |
| T_{smp.} = | 253°C |

Molecular formula: C₃₁H₃₃N₅O₄
Electrospray-mass spectrometry: m/z = 540 [M+H]⁺

| Element analysis: | | | |
|---|---|---|---|
| calculated | C 68.99 | H 6.16 | N 12.98 |
| found | C 68.32 | H 6.29 | N 12.85 |

In a second step, and in accordance with what what is disclosed in DE 102 33 500.1, the monoethanesulfonate salt of 3-Z-[1-(4-(N-((4-methyl-piperazin-1-yl)-methylcarbonyl)-N-methyl-amino)-anilino)-1-phenyl-methylene]-6-methoxycarbonyl-2-indolinone will be obtained as follows.

605 g (1.12 mol) 3-Z-[1-(4-(N-((4-methyl-piperazin-1-yl)-methylcarbonyl)-N-methyl-amino)-anilino)-1-phenyl-methylene]-6-methoxycarbonyl-2-indolinone are suspended in 9 litres methanol and heated to 50°C. 183.7 g (1.121 mol) of a 70% aqueous solution of ethanesulfonate is added. The resulting solution is cooled to 40°C and mixed with 4.5 litres ter-butylmethylether. Cristallisation occurs after a few minutes. In order to achieve a complete precipitation, the whole is mixed for 16 hours at room temperature. After cooling to a temperature of 10°C, the liquid is sucked up, the precipitate is washed with 2 litres ter-butylmethylether and vacuum dried at 40°C.

| | |
|---|---|
| Recovered product: | 638 g (87.6% of theoretical value) |
| T_{smp.} = | 305 ± 5°C (DSC 10K/min) |
| Purity (measured by HPLC): | 99.4% |
| Water content: | 1.0 bis 2.0% (KF) |

The monoethanesulfonate salt of 3-Z-[1-(4-(N-((4-methylpiperazin-1-yl)-methylcarbonyl)-N-methyl-amino)-anilino)-1-phenyl-methylene]-6-methoxycarbonyl-2-indolinone can be very easily dissolved in physiologically acceptable solubilization agents.

Additionally, this compound is orally bioavailable in mice. For example, the monoethanesulfonate salt of 3-Z-[1-(4-(N-((4-methyl-piperazin-1-yl)-methylcarbonyl)-N-methyl-amino)-anilino)-1-phenyl-methylene]-6-methoxycarbonyl-2-indolinone inhibits the VEGFR-2 kinase with an IC₅₀ of 21 nM and the proliferation of VEGF stimulated endothelial cells (HUVEC: IC₅₀= 9 nM, HSMEC: IC₅₀= 12 nM). The compound is highly selective when tested against a panel of 25 different kinases. Furthermore, FGFR-1 and PDGFRα, two members of the split kinase domain family of receptors important in angiogenic signaling, are additionally inhibited by this compound with IC₅₀'s of 69 nM and 59 nM respectively.

In conclusion, the monoethanesulfonate salt of 3-Z-[1-(4-(N-((4-methyl-piperazin-1-yl)-methylcarbonyl)-N-methylamino)-anilino)-1-phenyl-methylene]-6-methoxycarbonyl-2-indolinone is clearly a potent and orally available VEGFR-2 kinase inhibitor.

Further in vitro studies performed with the monoethanesulfonate salt of 3-Z-[1-(4-(N-((4-methyl-piperazin-1-yl)-methylcarbonyl)-N-methyl-amino)-anilino)-1-phenyl-methylene]-6-methoxycarbonyl-2-indolinone have shown that this specific compound has unexpected properties which makes it especially suitable for the treatment of the diseases in accordance with the present invention, especially when combined with a steroid, and more specifically with dexamethasone. Amongst these unexpected properties, the following are of particular relevance for the target indications: Tyrosine kinase inhibition of VEGFR1 to 3, FGFR1 and 3, PDGFR α; Inhibition of src-tyrosine kinase family members and potential inhibition of the proliferation of myeloma cells; Inhibition of the neo-angiogenesis induced by VEGF and bFGF; Inhibition of the paracrine IL-6 secretion; Inhibition of the cell contact mediated IL-6 secretion; Inhibition of the autocrine VEGF and bFGF effects; Direct induction of apoptosis on cell lines with t(4;14).

This specific compound appears to be further especially suitable for the treatment of multiple myeloma. The following recent findings constitute a line of evidence for the selection of this specific compound for this indication: Neovascularization parallels infiltration of bone marrow in a murine multiple myeloma model (Yaccoby et al., Blood 1998, Vol. 92(8), pp. 2908-2913) and in multiple myeloma patients undergoing progression (Vacca et al., Blood 1999, Vol. 93(9), pp. 3064-3073; Kumar et al., Blood 2002, Blood First Edition Paper, Prepublished Online October 17, 2002, DOI 10.1182/blood-2002-08-2441); VEGF has been shown to be a potent stimulus of angiogenesis (Toi et al., Lancet Oncol. 2001, Vol. 2, pp. 667-673); VEGF is expressed in and secreted by multiple myeloma cells (Dankbar et al., Blood 2000, Vol. 95(8), pp. 2630 -2636; Bellamy et al., Cancer Res. 1999, Vol. 59(3), pp. 728-33); VEGF induces IL-6 secretion from marrow stromal cells, which in turn augments VEGF expression from clonal plasma cells (Dankbar et al., Blood 2000, Vol. 95(8), pp. 2630 -2636); IL-6 is considered a major growth factor for multiple myeloma cells in vivo (Klein et al., Blood 1995, Vol. 85(4), pp. 863-872); IL-6 inhibits Dexamethasone-induced myeloma cell death (Hardin et al., Blood 1994, Vol. 84(9), pp. 3063-3070); VEGF induces proliferation and triggers migration of multiple myeloma cells (Podar et al., Blood 2001, Vol. 98(2), pp. 428-435); VEGF enhances osteoclastic bone resorption, which is a characteristic feature of multiple myeloma (Nakagawa et al., FEBS Lett. 2000, Vol. 473(2), pp. 161-164; Niida et al., J. Exp. Med. 1999, Vol. 190(2), pp. 293-298); FGFR3 induces proliferation, inhibits apoptosis and is involved in progression of myeloma cells (Chesi et al., Blood 2001, Vol. 97(3), pp. 729-736; Plowright et al., Blood 2000, Vol. 95(3), pp. 992-998); FGFR3 is dysregulated and constitutively activated in a subset of myeloma patients (Chesi et al., Blood 2001, Vol. 97(3), pp. 729-736; Chesi et al., Nat. Genet. 1997, Vol. 16(3), pp. 260-264); Src family kinases are involved in proliferative responses induced in myeloma (Ishikawa et al.; Blood 2002, Vol. 99(6), pp. 2172-2178).

With regard to all aspects of the invention, suitable protein tyrosine kinase receptor antagonists are also the active in vivo metabolites of protein tyrosine kinase receptor antagonists. For example, an active in vivo metabolite of the protein tyrosine kinase receptor antagonist 3-Z-[1-(4-(N-((4-methyl-piperazin-1-yl)-methylcarbonyl)-N-methyl-amino)-anilino)-1-phenyl-methylene]-6-methoxycarbonyl-2-indolinone may be the unesterified compound 3-Z-[1-(4-(N-((4-methyl-piperazin-1-yl)-methylcarbonyl)-N-methyl-amino)-anilino)-1-phenyl-methylene]-6-carbonyl-2-indolinone.

With regard to all aspects of the invention, suitable steroids for the combination treatment are meant to include in a non-limiting manner prednisone, prednisolone, methylprednisolone, dexamethasone, budenoside, fluocortolone and triamcinolone. The preferred steroid is dexamethasone.

Co-administration of the protein tyrosine kinase receptor antagonist and the steroid is meant to include administration sequential in time or simultaneous administration. For sequential administration, the protein tyrosine kinase receptor antagonist can be administered before or after administration of the steroid.

The active compounds can be administered orally, bucally, parenterally, by inhalation spray, rectally or topically, the oral administration being preferred. Parenteral administration may include subcutaneous, intravenous, intramuscular and intrasternal injections and infusion techniques.

The active compounds can be orally administered in a wide variety of different dosage forms, i.e., they may be formulated with various pharmaceutically acceptable inert carriers in the form of tablets, capsules, lozenges, troches, hard candies, powders, sprays, aqueous suspensions, elixirs, syrups, and the like. Such carriers include solid diluents or fillers, sterile aqueous media and various non-toxic organic solvents. Moreover, such oral pharmaceutical formulations can be suitably sweetened and/or flavoured by means of various agents of the type commonly employed for such purposes. In general, the compounds of this invention are present in such oral dosage forms at concentration levels ranging from about 0.5% to about 90% by weight of the total composition, in amounts which are sufficient to provide the desired unit dosages. Other suitable dosage forms for the compounds of this invention include controlled release formulations and devices well known to those who practice in the art.

For purposes of oral administration, tablets containing various excipients such as sodium citrate, calcium carbonate and calcium phosphate may be employed along with various disintegrants such as starch and preferably potato or tapioca starch, alginic acid and certain complex silicate, together with binding agents such as polyvinylpyrrolidone, sucrose, gelatine and acacia. Additionally, lubricating agents such as magnesium stearate, sodium lauryl sulfate and talc or compositions of a similar type may also be employed as fillers in soft and hard-filled gelatine capsules; included lactose or milk sugar as well as high molecular weight polyethylene glycols. When aqueous suspensions and/or elixirs are desired for oral administration, the essential active ingredient therein may be combined with various sweetening or flavouring agents, colouring matter or dyes and, if so desired, emulsifying agents and/or water, ethanol, propylene glycol, glycerine and various like combinations thereof.

For purposes of parenteral administration, solutions of the compounds in sesame or peanut oil or in aqueous propylene glycol may be employed, as well as sterile aqueous solutions of the corresponding pharmaceutically acceptable salts. Such aqueous solutions should be suitably buffered if necessary, and the liquid diluent rendered isotonic with sufficient saline or glucose. These particular aqueous solutions are especially suitable for intravenous, intramuscular and subcutaneous injection purposes. In this connection, the sterile aqueous media employed are readily obtained by standard techniques well known to those skilled in the art. For instance, distilled water is ordinarily used as the liquid diluent and the final preparation is passed through a suitable bacterial filter such as a sintered glass filter or a diatomaceous earth or unglazed porcelain filter. Preferred filters of this type include the Berkefeld, the Chamberland and the Asbestos Disk-Metal Seitz filter, wherein the fluid is sucked into a sterile container with the aid of a suction pump. The necessary steps should be taken throughout the preparation of these inject-able solutions to insure that the final products are obtained in a sterile condition.

For purposes of transdermal administration, the dosage form of the particular compound or compounds may include, by way of example, solutions, lotions, ointments, creams, gels, suppositories, rate-limiting sustained release formulations and devices therefor. Such dosage forms comprise the particular compound or compounds and may include ethanol, water, penetration enhancer and inert carriers such as gel-producing materials, mineral oil, emulsifying agents, benzyl alcohol and the like.

In accordance with one embodiment, the protein tyrosine kinase receptor antagonist, or its polymorph or pharmaceutically acceptable salt, may be administered in a daily dosage such that the plasma level of the active substance lies between 10 and 500 ng/ml for at least 12 hours of a 24 hours dosing interval.

In accordance with a further embodiment, the protein tyrosine kinase receptor antagonist, or its polymorph or pharmaceutically acceptable salt, may be administered in a daily dosage of between 2 mg and 20 mg /kg body weight.

In accordance with one embodiment, the steroid may be administered in a daily dosage of 5 to 500 mg.

## Claims

1. A method for the treatment of diseases involving cell proliferation, migration or apoptosis of myeloma cells, or angiogenesis, which method comprises co-administration of effective amounts of a protein tyrosine kinase receptor antagonist, or of a polymorph or pharmaceutically acceptable salt thereof, and of a steroid, to a person in need of such treatment.

2. The method in accordance with claim 1, wherein the protein tyrosine kinase receptor antagonist is an antagonist of at least one receptor selected from VEGFR 1 to 3, PDGFRα and β, FGFR1, 2 and 3, EGFR, HER2, IGF1R, HGFR, c-Kit.

3. The method in accordance with claim 1 or 2, wherein the protein tyrosine kinase receptor antagonist is also an antagonist of at least one complex of a cyclin dependent kinase with its specific cyclin or with a viral cyclin, an antagonist of a src-tyrosine kinase family member, or an inhibitor of the paracrine IL-6 secretion.

4. The method in accordance with any one of claims 1 to 3, wherein the protein tyrosine kinase receptor antagonist is 3-Z-[1-(4-(N-((4-methyl-piperazin-1-yl)-methylcarbonyl)-N-methyl-amino)-anilino)-1-phenyl-methylene]-6-methoxycarbonyl-2-indolinone , or a polymorph, metabolite or pharmaceutically acceptable salt thereof.

5. The method in accordance with any one of claims 1 to 4, wherein the protein tyrosine kinase receptor antagonist is the monoethanesulfonate salt of 3-Z-[1-(4-(N-((4-methyl-piperazin-1-yl)-methylcarbonyl)-N-methyl-amino)-anilino)-1-phenyl-methylene]-6-methoxycarbonyl-2-indolinone.

6. The method in accordance with any one of claims 1 to 5, wherein the steroid is selected from prednisone, prednisolone, methylprednisolone, dexamethasone, budenoside, fluocortolone and triamcinolone.

7. The method in accordance with any one of claims 1 to 6, wherein the steroid is dexamethasone.

8. The method in accordance with any one of claims 1 to 7, **characterised in that** the disease is an oncological disease.

9. The method in accordance with any one of claims 1 to 7, **characterised in that** the disease is a non-oncological disease selected from diabetic retinopathy, rheumatoid arthritis or psoriasis.

10. The method in accordance with any one of claims 1 to 8, **characterised in that** the disease is multiple myeloma.

11. The method in accordance with any of claims 1 to 10, **characterised in that** the protein tyrosine kinase antagonist, or its polymorph or pharmaceutically acceptable salt, is administered in a daily dosage such that the plasma level of the active substance lies between 10 and 500 ng/ml for at least 12 hours of a 24 hours dosing interval.

12. The method in accordance with any of claims 1 to 11, **characterised in that** the steroid is administered in a daily dosage of 5 to 500 mg.

13. Pharmaceutical combination for the treatment of diseases in which cell proliferation, migration or apoptosis of myeloma cells, or angiogenesis are involved, comprising the monoethanesulfonate salt of 3-Z-[1-(4-(N-((4-methyl-piperazin-1-yl)-methylcarbonyl)-N-methyl-amino)-anilino)-1-phenyl-methylene]-6-methoxycarbonyl-2-indolinone and a steroid, as a combined preparation for simultaneous, separate or sequential use in treatment of said diseases, optionally together with one or more pharmaceutically acceptable diluents and/or carriers.

14. The pharmaceutical combination in accordance with claim 13 wherein the steroid is selected from prednisone, prednisolone, methylprednisolone, dexamethasone, budenoside, fluocortolone and triamcinolone.

15. The pharmaceutical combination in accordance with claim 13 or 14 wherein the steroid is dexamethasone.

16. Use of a protein tyrosine kinase receptor antagonist, or a polymorph, metabolite or pharmaceutically acceptable salt thereof, in combination with a steroid, for the manufacture of a pharmaceutical combination preparation for the treatment of diseases in which cell proliferation or angiogenesis are involved, in a human or non-human mammalian body.

17. Use in accordance with claim 16, wherein the protein tyrosine kinase receptor antagonist is an antagonist of at least one receptor selected from VEGFR 1 to 3, PDGFRα and β, FGFR1, 2 and 3, EGFR, HER2, IGF1R, HGFR, c-Kit.

18. Use in accordance with claim 16 or 17, wherein the protein tyrosine kinase receptor antagonist is also an antagonist of at least one complex of a cyclin dependent kinase with its specific cyclin or with a viral cyclin, an antagonist of a src-tyrosine kinase family member, or an inhibitor of the paracrine IL-6 secretion.

19. Use in accordance with any one of claims 16 to 18, wherein the protein tyrosine kinase receptor antagonist is 3-Z-[1-(4-(N-((4-methyl-piperazin-1-yl)-methylcarbonyl)-N-methyl-amino)-anilino)-1-phenyl-methylene]-6-methoxycarbonyl-2-indolinone, or a polymorph, metabolite or pharmaceutically acceptable salt thereof.

20. Use in accordance with any one of claims 16 to 19, wherein the the protein tyrosine kinase receptor antagonist is the monoethanesulfonate salt of 3-Z-[1-(4-(N-((4-methyl-piperazin-1-yl)-methylcarbonyl)-N-methyl-amino)-anilino)-1-phenyl-methylene]-6-methoxycarbonyl-2-indolinone.

21. Use in accordance with any one of claims 16 to 20, wherein the steroid is selected from prednisone, prednisolone, methylprednisolone, dexamethasone, budenoside, fluocortolone and triamcinolone.

22. Use in accordance with any one of claims 16 to 21, wherein the steroid is dexamethasone.

23. Use in accordance with any one of claims 16 to 22, wherein the disease is an oncological disease.

24. Use in accordance with any one of claims 16 to 22, wherein the disease is a non-oncological disease selected from diabetic retinopathy, rheumatoid arthritis or psoriasis.

25. Use in accordance with any one of claims 16 to 23, **characterised in that** the disease is multiple myeloma.

26. A pharmaceutical combination preparation kit for the treatment of diseases involving cell proliferation, migration or apoptosis of myeloma cells, or angiogenesis, comprising a therapeutically effective amount of a protein tyrosine kinase receptor antagonist, or of a polymorph, metabolite or pharmaceutically acceptable salt thereof, and a therapeutically effective amount of a steroid, **characterised in that** the protein tyrosine kinase receptor antagonist is comprised within a first compartment and the steroid is comprised within a second compartment, such that the administration to a patient in need thereof can be simultaneous, separate or sequential.

27. The pharmaceutical combination preparation kit in accordance with claim 26, wherein the protein tyrosine kinase receptor antagonist is the monoethanesulfonate salt of 3-Z-[1-(4-(N-((4-methyl-piperazin-1-yl)-methylcarbonyl)-N-methylamino)-anilino)-1-phenyl-methylene]-6-methoxycarbonyl-2-indolinone and the steroid is dexamethasone.

28. The pharmaceutical combination preparation kit in accordance with claim 26 or 27, wherein the formulation of the active substances in each compartment is for oral administration.
